# EUROPEAN PATENT SPECIFICATION

(11) **EP 2 621 477 B1**
(45) Date of publication and mention of the grant of the patent: **19.03.2014**
(21) Application number: 11805527.6
(22) Date of filing: 23.12.2011
(51) Int. Cl.: A61K 9/20, A61K 9/28, A61K 31/606

(54) **ORAL PHARMACEUTICAL TABLET FOR CONTROLED RELEASE OF MESALAZINE AND PROCESS FOR OBTAINING IT**
ORALE PHARMAZEUTISCHE TABLETTE ZUR GESTEUERTEN ABGABE VON MESALAZIN UND VERFAHREN ZU DEREN ERHALT
COMPRIME PHARMACEUTIQUE ORAL POUR LA LIBERATION CONTROLEE DE LA MESALAZINE ET SON PROCEDE D'OBTENTION

(30) Priority: 27.12.2010 EP 10382355
(43) Date of publication of application: 07.08.2013
(73) Proprietor: Laboratorios Liconsa, S.A., 08028 Barcelona (ES)
(72) Inventor: LOECHES BLAS, David, E-08028 Barcelona (ES); VARAS FERNÁNDEZ-MOLINA, Roberto, E-08028 Barcelona (ES); MARTÍNEZ PÉREZ, Mercedes, E-08028 Barcelona (ES)
(74) Representative: Oficina Ponti, SLP
(86) International application number: PCT/EP2011/073983
(87) International publication number: WO 2012/089677

(56) References cited:
- WO-A1-03/090723
- WO-A2-03/011205
- WO-A2-2007/119177
- FR-A1- 2 692 484

## Description

### FIELD OF THE INVENTION

The present invention refers to an oral pharmaceutical tablet for controlled release of mesalazine as active ingredient, also named mesalamine or 5-amino salicylic acid.

The invention also refers to the process for obtaining said oral pharmaceutical tablet and to the oral pharmaceutical controlled release tablet of mesalazine for treating ulcerative colitis.

### BACKGROUND OF THE INVENTION

Inflammatory bowel disease (IBD) is a spectrum of chronic idiopathic inflammatory intestinal conditions. IDB causes significant GI symptoms that include diarrhea, abdominal pain, bleeding, anemia, and weight loss. IBD is also associated with a spectrum of extraintestinal mainfestations, including arthritis, ankylosing spondylitis, sclerosing cholangitis, uveitis, iritis, pyoderma gangrenosum, and erythema nodosum.

IBD is divided into two major subtypes: ulcerative colitis and Chron's disease. Ulcerative colitis is characterized by confluent mucosal inflammation of the colon starting at the anal verge and extending proxymally for a variable extent (eg. Proctitis, left-side colitis, or pancolitis). Chron's disease, by contrast, is characterized by transmural inflammation of any part of the GI tract but most comonly the area adjacent to the ileocecal valve.

First line therapy for mild-to-moderate ulcerative colitis generally involves mesalazine (5-aminosalicilic acid, or 5-ASA). Mesalazine is an aminosalicylate (5-aminosalicilic acid) with anti-inflammatory properties. Mesalazine is indicated for the induction of remission in patients with active, mild-to-moderate ulcerative colitis. Its chemical name is 5-amino-2-hydroxybenzoic acid and its structural formula is:

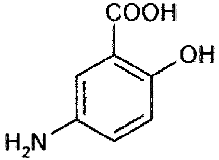

When mesalazine is administered orally, a large amount of the drug is absorbed from the upper gastrointestinal tract, causing systemic side effects. The mechanism of action of mesalazine is not fully understood, but appears to be topical.

Thus, the treatment of ulcerative colitis with mesalazine requires the specific delivery of the drug in the colon. Such specific delivery increases efficiency and enable reduction of the minimum effective dose. Various systems have been developed for colon-specific drug delivery. Enteric coating systems are the most commonly used for colonic drug delivery, due to the pH difference between small intestine and colon.

There are several modified-release mesalazine preparations currently commercialized for the treatment of mild to moderate acute exacerbations of UC, and maintenance of remission.

The development of efficacious modified release composition of mesalazine is hampered by the fact that this type of composition usually contains higher concentration of the active ingredient when compared with immediate release compositions on the same active. Another problem frequently encountered with conventional sustained release dosage forms is the inability to increase the residence time in an absorption window.

Patent application WO 03/011205-A discloses controlled release caplets of nifedipine comprising a matrix consisting of a 1:1 mixture of HPMC K15M and HPMC K100M, having viscosities of 13,275-24,780 mPa•s and 75,000-140000 mPa•s respectively, (see table 1) which provide a release of the active from the beginning of the administration with a zero order release of the active. After 5 hours from its administration, the 50% of the active is released. The active is completely released from the formulation after 10 hours from its administration.

Patent application WO 00/76481-A discloses controlled release tablets containing mesalazine as active ingredient comprising an inner lipophilic matrix with melting point below 90°C such us carnauba wax, in which the active ingredient is inglobated, an outer hydrophilic matrix such us hydroxypropylmethyl cellulose. The resulting tablets are then film coated with polymethacrylates to provide colonic delivery of the active.

WO 00/76481-A discloses the preparation of a tablet by melt granulation of the active ingredient with the lipophilic substance, to obtain granules which then are mixed with the hydrophilic excipients acting as hydrophilic matrix and subsequent tabletting or compression. The resulting tablets are then film coated with polymethacrylates to provide colonic delivery of the active.

However, low melting lipophilic materials blended with HPMC for achieving controlled or sustained release of an active ingredient have produced different results. Some of these systems have shown the failure to provide extended release of the drug, especially when the lipophilic material is used at high concentrations (≥10% w/w). Additionally, the melt granulation technique is expensive, time consuming and requires the use of special equipment. The melt granulation process consist of partially or completely melting the lipid excipient, then mixing with the active ingredient and other excipients and reducing the mixture to granules by chilling or congealing. During this process, the active ingredient is subjected to heat, and therefore if the temperature is not carefully controlled, the active ingredient can partially decompose. Additionally, subsequent cooling could induce phase transitions through the solid state or melt mechanism that could led to undesired variations in the release of the active.

The present invention provides an alternative oral pharmaceutically tablet that avoids the drawbacks of the above mentioned formulations.

### BRIEF DESCRIPTION OF THE INVENTION

The authors of the present invention have found that it is possible to provide a robust matrix system by combining different viscosity grades of HPMC to obtain the desired release features.

Surprisingly, it has been found that a hydrophilic matrix consisting of a mixture of hydroxypropylmethyl cellulose (HPMC) having a viscosity of less than 200 mPa•s in 2% aqueous solution and hydroxypropylmethyl cellulose (HPMC) having a viscosity higher than 200 mPa•s in 2% aqueous solution in a weight ratio between 10:1 to 1:10, overcome the drawbacks of the prior art formulations.

Advantageously, with the new oral pharmaceutical tablet for controlled release of mesalazine as active ingredient an initial burst release of the active ingredient from the matrix is avoided and a desired colonic release profile of mesalazine as active ingredient is achieved.

The invention also provides a process for the preparation of said oral pharmaceutical tablet designed for the sustained or controlled release of mesalazine as active ingredient in the tablet.

At least another object of the invention relates to said oral pharmaceutical tablet for the treatment of ulcerative colitis.

### FIGURES

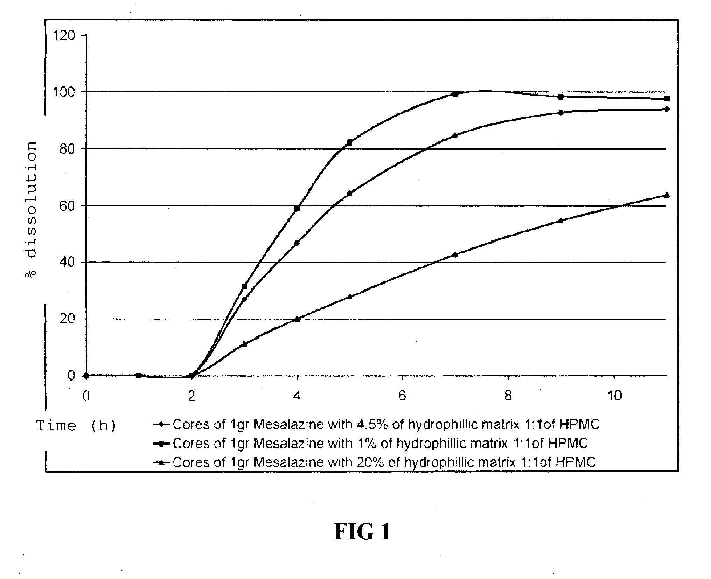

Figure 1 shows the different dissolution profiles obtained with the cores of controlled release of mesalazine before cover them with the outer cover layer in order to obtain the tablet according to the present invention. The symbol ■ shows the dissolution profile of cores containing 1g of Mesalazine with 1% by weight based on the total weight of the tablet of a hydrophilic matrix consisting of hydroxypropylmethyl cellulose (HPMC) having a viscosity of less than 200 mPa•s in 2% aqueous solution and hydroxypropylmethyl cellulose (HPMC) having a viscosity of higher than 200 mPa•s in 2% aqueous solution (example 2). The symbol ◆ shows the dissolution profile of cores containing 1g of Mesalazine with 4.5% by weight based on the total weight of the tablet of a hydrophilic matrix consisting of hydroxypropylmethyl cellulose (HPMC) having a viscosity of less than 200 mPa•s in 2% aqueous solution and hydroxypropylmethyl cellulose (HPMC) having a viscosity of higher than 200 mPa•s in 2% aqueous solution (Example 1). The symbol A shows the dissolution profile of cores containing 1g of Mesalazine with 20% by weight based on the total weight of the tablet of a hydrophilic matrix consisting of hydroxypropylmethyl cellulose (HPMC) having a viscosity of less than 200 mPa•s in 2% aqueous solution and hydroxypropylmethyl cellulose (HPMC) having a viscosity of higher than 200 mPa•s in 2% aqueous solution (example 3).

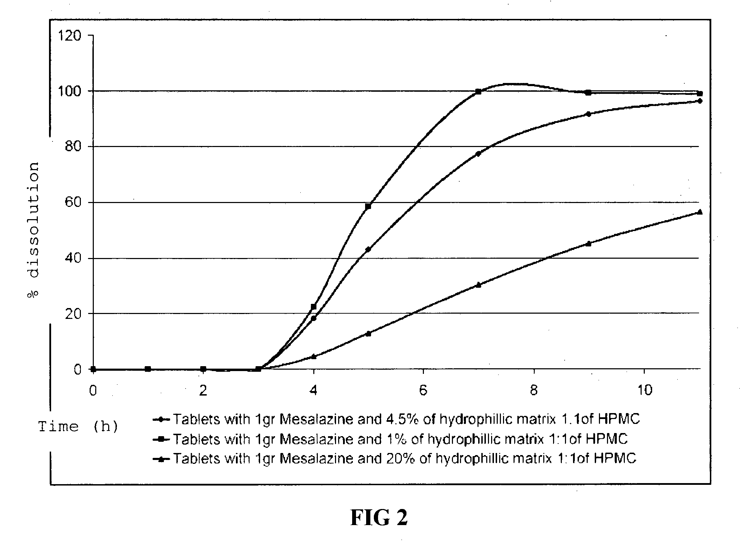

Figure 2 shows the different dissolution profiles obtained with the oral pharmaceutical tablet of controlled release of mesalazine according to the present invention, which tablets comprise the cores of figure 1. The meaning of the symbols are the same than in figure 1.

From the two figures, it can be observed that both cores and tablets maintain the same dissolution profile of mesalazine. Further, figure 1 demonstrates that the used hydrophilic matrix in the core of the tablet according to the present invention produces the dissolution effect with the desired profile of controlled release of mesalazine.

### DETAILED DESCRIPTION OF THE INVENTION

The present invention provides an oral pharmaceutical tablet of controlled release of mesalazine or a pharmaceutically acceptable salt thereof as active ingredient wherein said tablet comprises a core and a gastro-resistant outer coating.

As used herein, "controlled release of mesalazine" means a dosage form in which the release of mesalazine is modified (or sustained) over a period of time compared to an immediate release formulation.

According to the present invention, mesalazine may be in any crystalline or amorphous form.

Thus, the invention provides an oral pharmaceutical tablet for controlled release of mesalazine or a pharmaceutically acceptable salt thereof as active ingredient with a core and a gastro-resistant outer coating, characterized in that
said core comprises:
i) mesalazine from 40% to 90%, preferably from 50% to 90%, more preferably from 60% to 80% by weight based on the total weight of the tablet; and
ii) a hydrophilic matrix consisting of hydroxypropylmethyl cellulose (HPMC) having a viscosity of less than 200 mPa•s in 2% aqueous solution and hydroxypropylmethyl cellulose (HPMC) having a viscosity higher than 200 mPa•s in 2% aqueous solution in a weight ratio between 10:1 to 1:10, preferably in a weight ratio of 1:1, which is present from 1% up to 20%, preferably from 1% to 15%, more preferably from 2% to 10%, and still more preferably from 3% to 5% by weight based on the total weight of the tablet;
   and said gastro-resistant outer coating comprises:
iii) a pH-dependent release polymer present in an amount from 15% to 75% by weight of the outer cover layer of the tablet, wherein said outer cover layer is present from 5% to 25%, preferably from 10% to 20% based on the total weight of the tablet.

Surprisingly, the two viscosity grade HPMC polymers with these ratio provides hydrophilic matrices with improved physical features that exhibit similar dissolution profiles at different agitation speeds.

Suitable HPMC polymers include those sold under the trademark METHOCEL® (Dow Chemical Corporation) or the trademark METOLOSE® (Shin-Etsu).The polymer chemistry and viscosity of different families of Methocel HPMC can be found in table 1. The number designation after each letter denotes the viscosity of a 2 wt% aqueous solution at 25°C.

**Table 1**

| **METHOCEL™ Product** | **Viscosity of 2% solution in water, mPa·s (USP/EP/JP)** |
|---|---|
| METHOCEL™ E3 Premium LV | 2.4 - 3.6 |
| METHOCEL™ E5 Premium LV | 4.0 - 6.0 |
| METHOCEL™ E6 Premium LV | 4.8 - 7.2 |
| METHOCEL™ E15 Premium LV | 12 - 18 |
| METHOCEL™ E50 Premium LV | 40 - 60 |
| METHOCEL™ E4M Premium | 2663 - 4970 |
| METHOCEL™ E10M Premium CR | 9525 - 17780 |
| METHOCEL™ F50 Premium | 40 - 60 |
| METHOCEL™ F4M Premium | 2663 - 4970 |
| METHOCEL™ K3 Premium LV | 2.4 - 3.6 |
| METHOCEL™ K100 Premium LV | 80 - 120 |
| METHOCEL™ K4M Premium | 2663 - 4970 |
| METHOCEL™ K15M Premium | 13275 - 24780 |
| METHOCEL™ K100M Premium | 75000 - 140000 |

In a preferred embodiment the hydrophilic matrix consist of a mixture of viscosity 80-120 mPa•s of 2% solution in water (Methocel K100 Premium LV) and viscosity 2663 - 4970 mPa•s of 2% solution in water (METHOCEL™ K4M Premium) in a weight ratio of 1:1.

The tablet(s) of the formulation may be coated with one or more layers of a pH-dependent release polymer.

As used herein, the term "pH-dependent release polymer" also named enteric polymer means a polymer that is insoluble at the highly acidic pH found in the stomach, but dissolves rapidly at a less acidic (relatively more basic) pH. According to the invention, the pH-dependent release polymer will not dissolve in the acidic juices of the stomach (pH ∼3), but will do it in the higher pH environment present in the small intestine as at a pH above 5.5 or in the colon as at a pH above 7.0.

The pH-dependent release polymer is selected such that mesalazine will be released at about the time that the dosage form reaches the inlet between the small intestine and the colon, or thereafter in the colon. The selection is based upon the pH profile of the small intestine and colon. The pH of the small intestine gradually increases from about 5 to 5.5 in the duodenal bulb to about 7.2 in the distal portions of the small intestine (ileum). The pH drops significantly at the ileocecal junction to about 6.3 and very gradually increases to about 7 in the left or descending colon.

The authors have found that the use of a high-viscosity grade HPMC as a matrix former allows swelling the core upon absorption of the gastric fluid and the gradual erosion over a time period of hours, since it increases the gel strength. The addition of low viscosity grade HMPC avoids an initial burst release of the active ingredient from the matrix, thus allowing consistent erosion. The erosion process is simultaneously initiated with the swelling process upon contact of the surface of the dosage form with the gastric fluid. Erosion reflects the dissolution of the polymer beyond the formation of the gel-solution interface, where the polymer has become sufficiently diluted that the active ingredient can be transported away from the dosage form by diffusion or convention.

The hydrophilic matrix according to the invention provides the desired colonic release profile, providing sustained release of the active within the desired absorption window.

With the oral pharmaceutical tablet of the present invention a release of mesalazine is achieved in the colon for which pharmacological activity is desired at time significantly delayed from the time of peroral administration. Taking into account the route of a tablet from its peroral administration to the colon, the oral pharmaceutical tablet of the present invention provides an adequate colonic release profile.

Preferred pH-dependent release polymers are those, which remain intact in the lower pH environs of the stomach and small intestine, but begin to dissolve in an aqueous solution at a pH above 6.3, preferably between 6.8 to 7.2. Preferred enteric polymer are selected from poly(methacrylic acid, methyl methacrylate) 1:2 (Eudragit^{®} S), and mixtures of poly(methacrylic acid, methyl methacrylate) 1:1 (Eudragit^{®} L) and poly(methacrylic acid, methyl methacrylate) 1:2 (Eudragit^{®} S) in a ratio of about 1:10 to about 1:1, preferably about 1:5 to about 1:3. Especially preferred are Eudragit^{®} S and Eudragit^{®} L or mixtures thereof. Especially preferred is a mixture 1:10 to 10:1 of Eudragit^{®} S and Eudragit^{®} L.

In a preferred embodiment, the pH-dependent release polymer is present in an amount from 15% to 75% by weight of the outer cover layer of the tablet.

Preferably, outer cover layer is present from 5% to 25% based on the total weight of the tablet formulation, preferably from 10% to 20%.

Optionally, the core further comprises a filler, a binding agent, an antiadherent agent, a lubricant and a disintegrant as a pharmaceutically acceptable excipient. Optionally, the outer cover layer of the tablet further comprises an antiadherent, a plasticizer and a colorant as a pharmaceutically acceptable excipient.

Preferably, the tablet(s) of the invention comprises a filler. Materials commonly used as fillers include, but are not limited to, dextrose, lactose, lactose monohydrate, fructose, mannitol, microcrystalline cellulose, starch, pregelatinized starch, powdered cellulose, silicied cellulose, sorbitol, sucrose and talc, or mixtures thereof. Preferred filler is microcrystalline cellulose. The filler may be present in an amount from 0.5% to 10% by weight, preferably from 0.5% to 8% by weight, and more preferably from 1% to 5% by weight of the total weight of the tablet.

Preferably, the tablet(s) of the invention comprises a binding agent or binder. Materials commonly used as binders include, but are not limited to, polyvinylpyrrolidone, hydroxypropylcellulose, hydroxypropylnethylcellulose, hydroxyethylcellulose, methylcellulose, sodium carboxymethyl cellulose, calcium carboxymethylcellulose, calcium carboxymethyl cellulose and/or mixtures thereof. Preferred binding agent is polyvinylpyrrolidone. The binder may be present in an amount from 0.1% to 10% by weight, preferably from 0.5% to 9% by weight, and more preferably from 1% to 7.5% by weight of the total weight of the tablet.

Preferably, the tablet formulation according to the invention comprises an antiadherent agent in the core. The outer cover layer also may comprise an antiadherent agent to eliminate sticking during the film coating process.

Materials commonly used as antiadherent include, but are not limited to, colloidal silicon dioxide and talc, being preferable the colloidal silicon dioxide (aerosil) when is used in the core and talc when is used in the outer cover layer.

The antiadherent agent is present in the core in an amount of from 0.1% to 5% by weight, preferably from 0.1% to 3.5% by weight, more preferably from 0.1% to 1.5% by weight based on the total weight of the tablet, and up to 30% by weight, preferably from 5% to 30% by weight based on the total weight of the outer cover layer.

Preferably, the tablet formulation according to the invention optionally comprises a lubricant. Materials commonly used as lubricant include, but are not limited to, stearic acid, and stearic acid salts such as magnesium stearate. A preferred lubricant is magnesium stearate. The lubricant may be present 0.1% to 5% by weight, preferably from 0.1% to 3% by weight based on the total weight of the tablet.

Preferably, the tablet formulation according to the invention optionally comprises a disintegrant. Materials commonly used as disintegrant include, but are not limited to, starch, pregelatinized starch, low-substituted hydroxypropylcellulose, sodium carboxymethyl cellulose, crospovidone, sodium croscarmellose and/or mixtures thereof. Preferably, the disintegrant agent is pregelatinized starch.

The disintegrant agent is present in the core of the tablet(s) from 1% to 10% by weight, preferably from 1% to 8% by weight, more preferably from 2.5% to 7.5% by weight based on the total weight of the tablet.

Preferably, the outer cover layer optionally comprises a plasticizer to provide homogeneous film mixtures. Preferred plasticizer includes triethyl citrate, polyethylene glycol, or dibutyl sebacate, preferably triethyl citrate. The plasticizer agent may be present in an amount up to 20%, preferably from 5% to 20% y weight based on the total weight of the outer cover layer.

The tablet(s) of the invention includes one or more pharmaceutically acceptable excipients. All such excipients must be "pharmaceutically acceptable" in the sense of being compatible with the other ingredients of the pharmaceutical composition and not injurious to the patient. Pharmaceutically acceptable excipients may include colours, flavours e.g. menthol, sweeteners e.g. mannitol, preservatives, stabilisers, antioxidants and any other excipients known to those skilled in the art.

In a preferred embodiment, the pharmaceutical tablet according to the invention comprises a core comprising from 40% to 90% by weight of mesalazine, from 1% to 20% of a 1:10 to 10:1 weight ratio mixture of hydroxypropylmethyl cellulose (HPMC) having a viscosity of less than 200 mPa•s in 2% aqueous solution and hydroxypropylmethyl cellulose (HPMC) having a viscosity higher than 200 mPa•s in 2% w/w aqueous solution, from 0.5% to 10% of microcrystalline cellulose, from 0.1% to 10% by weight of polyvinyl pyrrolidone, and from 1% to 10% by weight of pregelatinized starch, and the outer cover layer comprising a pH-dependent release polymer and one or more pharmaceutically acceptable excipients.

In a more preferred embodiment, the pharmaceutical tablet according to the invention comprises a core comprising a core comprising from 50% to 90% by weight of mesalazine, from 2% to 10% of a 1:10 to 10:1 weight ratio mixture of hydroxypropylmethyl cellulose (HPMC) having a viscosity of less than 200 mPa•s in 2% aqueous solution and hydroxypropylmethyl cellulose (HPMC) having a viscosity higher than 200 mPa•s in 2% w/w aqueous solution, from 0.5% to 8% of microcrystalline cellulose, from 0.5% to 9% by weight of polyvinyl pyrrolidone, and from 2% to 8% by weight of pregelatinized starch, and the outer cover layer comprising a pH-dependent release polymer and one or more pharmaceutically acceptable excipients.

In a more preferred embodiment, the pharmaceutical tablet according to the invention comprises a core comprising a core comprising from 60% to 80% by weight of mesalazine, from 3% to 5% of a 1:10 to 10:1 weight ratio mixture of hydroxypropylmethyl cellulose (HPMC) having a viscosity of less than 200 mPa•s in 2% aqueous solution and hydroxypropylmethyl cellulose (HPMC) having a viscosity higher than 200 mPa•s in 2% w/w aqueous solution, from 1% to 5% of microcrystalline cellulose, from 1% to 7.5% by weight of polyvinyl pyrrolidone, and from 2.5% to 7.5% by weight of pregelatinized starch, and the outer cover layer comprising a pH-dependent release polymer and one or more pharmaceutically acceptable excipients.

The second aspect of the present invention is to provide a process for obtaining the oral pharmaceutical tablet according to the first aspect of the invention, said process comprising the steps of:
a) blending mesalazine with the hydrophilic matrix consisting of hydroxypropylmethyl cellulose (HPMC) having a viscosity of less than 200 mPa•s in 2% aqueous solution and hydroxypropylmethyl cellulose (HPMC) having a viscosity higher than 200 mPa•s in a weight ratio between 1:10 to 10:1, and with the disintegrant and the binder, if they are present, to obtain a blended mixture;
b) granulating the blended mixture obtained in step a) with water or with an aqueous solution of binder, previously prepared, when the binder is present in step a);
c) drying the granulate obtained in step b);
d) lubricating, if a lubricant is present, the dried granules and compressing it to obtain the core; and
e) preparing an aqueous dispersion which comprises the pH-dependent polymer with the pharmaceutically acceptable excipients, and coating the core to obtain the outer cover layer, and thus the tablet.

The method used for coating the tablet(s) may be any conventional method known to a person skilled in the art.

Advantageously, in step b) an aqueous solution of polyvinyl pyrrolidone is prepared; in step c) the drying is carried out in a fluid bed dryer; in step d) a lubricant is added for lubricating the dried granules and, then, the blend compressed to obtain a core, and in step e) an aqueous dispersion of an alcoholic solution of an antiadherent agent and the pH-dependent polymer and a plastisizer is prepared for coating the core obtaining an outer cover layer.

In another aspect, the present invention relates to the tablets according to the invention for treating ulcerative colitis.

The following examples are given to illustrate the invention in a sufficiently complete manner.

### EXAMPLES

### Example 1

| **Tablet component** | **(mg/comp.)** | **Function** |
|---|---|---|
| **Tablet Core** | | |
| Mesalazine | 1000.00 | API |
| HPMC Methocel K4M Premium | 31.67 | Hydrophilic matrix |
| HPMC Methocel K100 LV Premium | 31.67 | Hydrophilic matrix |
| Polivinylpirrolidone K30 | 25.33 | Binder |
| Pregelatinized starch | 60.83 | Disintegrant |
| Cellulose Microcrystalline | 40.17 | Filler |
| Aerosil 200 | 18.67 | Antiadherent |
| Magnesium stearate | 10.00 | Lubricant |

| **Tablet component** | **(mg/comp.)** | **Function** |
|---|---|---|
| **Tablet coating** | | |
| Talc | 29.14 | antiadherent |
| Methacrylic acid/methyl methacrylate copolymer 1:2 (Eudragit^{®} S) | 10.02 | Controlled Release Polymer |
| Methacrylic acid/methyl methacrylate copolymer 1:1 (Eudragit^{®} L) | 90.18 | Controlled Release Polymer |
| Triethyl citrate | 80.16 | Plasticizer |
| Titanium dioxide | 0.5 | Colorant |
| Red iron oxide | 1.5 | Colorant |

Mesalazine, HPMC Methocel K4M Premium, HPMC Methocel K100 LV Premium and pregelatinized starch are mixed and subsequently granulated with a polyvinyl pyrrolidone solution (15%) in purified water.

The granules are dried in a fluid bed dryer. The dried granules are passed through suitable mesh sieve. These granules and mixed with colloidal silicon dioxide and microcrystalline cellulose and lubricated with magnesium stearate.

The final blend is then compressed.

The tablets are coated with the gastro-resistant coating suspension (alcoholic solution of talc, Eudragit S 100, Eudragit L100, and triethylcitrate).

### Examples 2 to 3

Using a procedure similar to that described in Example 1, tablets containing 1000 mg of mesalazine and the different amounts of the HPMC mixture shown in Table 1 were prepared.

| Example | HPMC Methocel K4M : HPMC Methocel K100 LV 1:1 mixture (% by weight)* |
|---|---|
| 2 | 1 |
| 3 | 20 |

| | |
|---|---|
| * The weight percent refers to the total weight of the tablet(s). | |

Composition of tablets produced according to examples 2 and 3 is shown below:

| **Tablet component** | **Example 2 (mg/comp.)** | **Example 3 (mg/comp.)** |
|---|---|---|
| **Tablet Core** | | |
| Mesalazine | 1000,00 | 1000,0 |
| HPMC Methocel K4M Premium | 6,09 | 121,8 |
| HPMC Methocel K100 LV Premium | 6,09 | 121,8 |
| Polivinylpirrolidone K30 | 25,33 | 25,30 |
| Pregelatinized starch | 60,83 | 60,80 |
| Cellulose Microcrystalline | 46,83 | 46,80 |
| Aerosil 200 | 18,67 | 18,67 |
| Magnesium stearate | 10,00 | 10,00 |

| **Tablet component** | **(mg/comp.)** | **(mg/comp.)** |
|---|---|---|
| **Tablet coating** | | |
| Talc | 27,92 | 33,43 |
| Methacrylic acid/methyl methacrylate copolymer 1:2 (Eudragit® S) | 9,60 | 11,49 |
| Methacrylic acid/methyl methacrylate copolymer 1:1 (Eudragit® L) | 86,41 | 103,45 |
| Triethyl citrate | 76,81 | 91,96 |
| Titanium dioxide | 0,48 | 0,57 |
| Red iron oxide | 1,44 | 1,72 |

### Dissolution method

For all examples, the tablets were tested for dissolution of mesalazine at USP Type II apparatus. Dissolution medium: 2 hours in HCl, 1 hour at pH 6,4 and 9 hours at pH 7.2.

The dissolution profile obtained for the formulations described in examples 1 to 3 is disclosed in Figure 1.

## Claims

1. An oral pharmaceutical tablet for controlled release of mesalazine or a pharmaceutically acceptable salt thereof as active ingredient with a core and a gastro-resistant outer coating, **characterized in that** said core comprises:
i) mesalazine from 40% to 90% by weight based on the total weight of the tablet; and
ii) a hydrophilic matrix consisting of hydroxypropylmethyl cellulose (HPMC) having a viscosity of less than 200 mPa•s in 2% aqueous solution and hydroxypropylmethyl cellulose (HPMC) having a viscosity higher than 200 mPa•s in 2% aqueous solution in a weight ratio between 10:1 to 1:10, being said hydrophilic matrix present from 1% up to 20% by weight based on the total weight of the tablet;
and **in that** said gastro-resistant outer coating comprises:
iii) a pH-dependent release polymer,
being said outer cover layer present from 5% to 25% based on the total weight of the tablet.

2. An oral pharmaceutical tablet according to claim 1, wherein mesalazine is present from 50% to 90%, preferably from 60% to 80% by weight based on the total weight of the tablet;

3. An oral pharmaceutical tablet according to claim 1, wherein the hydrophilic matrix is present from 1% to 15%, preferably from 2% to 10%, and more preferably from 3% to 5% by weight based on the total weight of the tablet.

4. An oral pharmaceutical tablet according to claim 1, wherein the outer cover layer is present from 10% to 20% based on the total weight of the tablet.

5. An oral pharmaceutical tablet according to claim 1, wherein the hydrophilic matrix consisting of hydroxypropylmethyl cellulose (HPMC) having a viscosity of less than 200 mPa•s in 2% aqueous solution and hydroxypropylmethyl cellulose (HPMC) having a viscosity higher than 200 mPa•s in 2% aqueous solution in a weight ratio of 1:1.

6. An oral pharmaceutical tablet according to claim 1, wherein the hydrophilic matrix is present from 1% to 15%, preferably from 2% to 10%, and more preferably from 3% to 5% by weight based on the total weight of the tablet.

7. An oral pharmaceutical tablet according to claim 1, wherein the pH-dependent release polymer is present in an amount from 15% to 75% by weight of the outer cover layer of the tablet.

8. An oral pharmaceutical tablet according to claim 1, wherein the core further comprises a pharmaceutically acceptable excipient selected from filler, a binder, an antiadherent agent, a lubricant or a disintegrant.

9. An oral pharmaceutical tablet according to claim 1, wherein the outer cover layer of the tablet further comprises a pharmaceutically acceptable excipient selected from an antiadherent, a plasticizer or a colorant.

10. An oral pharmaceutical tablet according to claim 8, wherein the filler is present in an amount from 0.5% to 10%, preferably from 0.5% to 8%, and more preferably from 1% to 5% by weight of the total weight of the tablet.

11. An oral pharmaceutical tablet according to claim 8, wherein the binder is present in an amount from 0.1% to 10%, preferably from 0.5% to 9%, and more preferably from 1% to 7.5% by weight of the total weight of the tablet.

12. An oral pharmaceutical tablet according to claim 8 and 9, wherein the antiadherent agent is present in an amount of from 0.1% to 5%, preferably from 0.1% to 3.5%, more preferably from 0.1% to 1.5% by weight based on the total weight of the tablet, and up to 30%, preferably from 5% to 30% by weight based on the total weight of the outer cover layer.

13. An oral pharmaceutical tablet according to claim 8, wherein the lubricant is present from 0.1% to 5%, preferably from 0.1% to 3% by weight based on the total weight of the tablet.

14. An oral pharmaceutical tablet according to claim 8, wherein the disintegrant agent is present from 1% to 10%, preferably from 1% to 8%, more preferably from 2.5% to 7.5% by weight based on the total weight of the tablet.

15. An oral pharmaceutical controlled release tablet according to claim 9, wherein the plasticizer agent is present in an amount up to 20%, preferably from 5% to 20% by weight based on the total weight of the outer cover layer.

16. Process for obtaining the oral pharmaceutical tablet according to any of claims 1 to 15, **characterized in that** the following steps are carried out:
a) blending mesalazine with the hydrophilic matrix consisting of hydroxypropylmethyl cellulose (HPMC) having a viscosity of less than 200 mPa•s in 2% aqueous solution and hydroxypropylmethyl cellulose (HPMC) having a viscosity higher than 200 mPa•s in a weight ratio between 1:10 to 10:1, and with the disintegrant and the binder, if they are present, to obtain a blended mixture;
b) granulating the blended mixture obtained in step a) with water or with an aqueous solution of binder, previously prepared, when the binder is present in step a);
c) drying the granulate obtained in step b);
d) lubricating, if a lubricant is present, the dried granules and compressing it to obtain the core; and
e) preparing an aqueous dispersion which comprises the pH-dependent polymer with the rest of pharmaceutically acceptable excipients, and coating the core to obtain the outer cover layer, and thus the tablet.

17. Process according to claim 16, wherein in step b) an aqueous solution of polyvinyl pyrrolidone is prepared.

18. Process according to claim 16, wherein in step c) the drying is carried out in a fluid bed dryer;

19. Process according to claim 16, wherein in step d) a lubricant is added for lubricating the dried granules and, then, the blend compressed to obtain a core.

20. Process according to claim 16, wherein in step e) an aqueous dispersion of an alcoholic solution of an antiadherent agent and the pH-dependent polymer and a plastisizer is prepared for coating the core and then obtaining the outer cover layer.

21. The oral pharmaceutical tablet for controlled release of mesalazine or a pharmaceutically acceptable salt thereof according to any of claims 1 to 15 for treating ulcerative colitis.

## Patentansprüche

1. Orale pharmazeutische Tablette zur gesteuerten Abgabe von Mesalazin oder eines pharmazeutisch akzeptierbaren Salzes davon als aktives Ingrediens mit einem Kern und einer magenresistenten äußeren Schicht, **dadurch gekennzeichnet, dass** der Kern aufweist:
(i) Mesalazin in einer Menge von 40 bis 90 Gew.-% basierend auf dem Gesamtgewicht der Tablette; und
(ii) eine hydrophile Matrix bestehend aus Hydroxypropylmethyl-cellulose (HPMC) mit einer Viskosität von weniger als 200 mPa•s in 2 % wässriger Lösung und Hydroxypropylmethyl-cellulose (HPMC) mit einer Viskosität von mehr als 200 mPa•s in 2 % wässriger Lösung in einem Gewichtsverhältnis von zwischen 10:1 und 1:10, wobei die hydrophile Matrix in einer Menge von 1 bis 20 Gew.-% basierend auf dem Gesamtgewicht der Tablette vorhanden ist;
und dass die magenresistente äußere Schicht aufweist:
(iii) ein pH-abhängiges Abgabe-Polymer,
wobei die äußere Schutzschicht in einer Menge von 5 bis 25 Gew.-% basierend auf dem Gesamtgewicht der Tablette vorhanden ist.

2. Orale pharmazeutische Tablette nach Anspruch 1, bei der Mesalazin in einer Menge von 50 bis 90 Gew.-%, vorzugsweise von 60 bis 80 Gew.-%, basierend auf dem Gesamtgewicht der Tablette, vorhanden ist.

3. Orale pharmazeutische Tablette nach Anspruch 1, bei der die hydrophile Matrix in einer Menge von 1 bis 15 Gew.-%, vorzugsweise von 2 bis 10 Gew.-%, und bevorzugt von 3 bis 5 Gew.-%, basierend auf dem Gesamtgewicht der Tablette, vorhanden ist.

4. Orale pharmazeutische Tablette nach Anspruch 1, bei der die äußere Schutzschicht in einer Menge von 10 bis 20 Gew.-% basierend auf dem Gesamtgewicht der Tablette vorhanden ist.

5. Orale pharmazeutische Tablette nach Anspruch 1, bei der die hydrophile Matrix aus Hydroxypropylmethyl-cellulose (HPMC) mit einer Viskosität von weniger als 200 mPa•s in 2 % wässriger Lösung und Hydroxypropylmethyl-cellulose (HPMC) mit einer Viskosität von mehr als 200 mPa•s in 2 % wässriger Lösung in einem Gewichtsverhältnis von 1:1 besteht.

6. Orale pharmazeutische Tablette nach Anspruch 1, bei der die hydrophile Matrix in einer Menge von 1 bis 15 Gew.-%, vorzugsweise von 2 bis 10 Gew.-%, und bevorzugt von 3 bis 5 Gew.-%, basierend auf dem Gesamtgewicht der Tablette, vorhanden ist.

7. Orale pharmazeutische Tablette nach Anspruch 1, bei der das pH-abhängige Abgabe-Polymer in einer Menge von 15 bis 75 Gew.-% der äußeren Schutzschicht der Tablette vorhanden ist.

8. Orale pharmazeutische Tablette nach Anspruch 1, bei der der Kern weiterhin ein pharmazeutisch akzeptierbares Vehikel aufweist, das ausgewählt ist aus einer Gruppe bestehend aus Füllmittel, Bindemittel, Antihaftmittel, Schmiermittel und Sprengmittel.

9. Orale pharmazeutische Tablette nach Anspruch 1, bei der die äußere Schutzschicht der Tablette weiterhin ein pharmazeutisch akzeptierbares Vehikel aufweist, das ausgewählt ist aus einer Gruppe bestehend aus Antihaftmittel, Plastiziermittel und Farbmittel.

10. Orale pharmazeutische Tablette nach Anspruch 8, bei der das Füllmittel in einer Menge von 0,5 bis 10 Gew.-%, vorzugsweise von 0,5 bis 8 Gew.-%, und bevorzugt von 1 bis 5 Gew.-% des Gesamtgewichts der Tablette vorhanden ist.

11. Orale pharmazeutische Tablette nach Anspruch 8, bei der das Bindemittel in einer Menge von 0,1 bis 10 Gew.-%, vorzugsweise von 0,5 bis 9 Gew.-%, und bevorzugt von 1 bis 7,5 Gew.-% des Gesamtgewichts der Tablette vorhanden ist.

12. Orale pharmazeutische Tablette nach den Ansprüchen 8 und 9, bei der das Antihaftmittel in einer Menge von 0,1 bis 5 Gew.-%, vorzugsweise von 0,1 bis 3,5 Gew.-%, bevorzugt von 0,1 bis 1,5 Gew.-%, basierend auf dem Gesamtgewicht der Tablette, und bis zu 30 Gew.-%, vorzugsweise von 5 bis 30 Gew.-%, basierend auf dem Gesamtgewicht der Schutzschicht, vorhanden ist.

13. Orale pharmazeutische Tablette nach Anspruch 8, bei der das Schmiermittel in einer Menge von 0,1 bis 5 Gew.-%, vorzugsweise von 0,1 bis 3 Gew.-%, basierend auf dem Gesamtgewicht der Tablette, vorhanden ist.

14. Orale pharmazeutische Tablette nach Anspruch 8, bei der das Sprengmittel in einer Menge von 1 bis 10 Gew.-%, vorzugsweise von 1 bis 8 Gew.-%, und bevorzugt von 2,5 bis 7,5 Gew.-%, basierend auf dem Gesamtgewicht der Tablette, vorhanden ist.

15. Orale pharmazeutische Tablette zur gesteuerten Abgabe nach Anspruch 9, bei der das Plastiziermittel in einer Menge von bis zu 20 Gew.-%, vorzugsweise von 5 bis 20 Gew.-%, basierend auf dem Gesamtgewicht der äußeren Schutzschicht, vorhanden ist.

16. Verfahren zum Erhalt der oralen pharmazeutischen Tablette nach einem der Ansprüche 1 bis 15, **dadurch gekennzeichnet, dass** die folgenden Schritte ausgeführt werden:
a) Mischen des Mesalazin mit der hydrophilen Matrix bestehend aus Hydroxypropylmethyl-cellulose (HPMC) mit einer Viskosität von weniger als 200 mPa•s in 2 % wässriger Lösung und aus Hydroxypropylmethyl-cellulose (HPMC) mit einer Viskosität von mehr als 200 mPa•s in 2 % in einem Gewichtsverhältnis von zwischen 1:10 und 10:1, und mit dem Sprengmittel und dem Bindemittel, sofern diese vorhanden sind, um ein Gemisch zu erhalten;
b) Granulieren des in Schritt a) erhaltenen Gemisches mit Wasser oder mit einer wässrigen Lösung aus Bindemittel, welches zuvor zubereitet wurde, wenn das Bindemittel in Schritt a) vorhanden ist;
c) Trocknen des in Schritt b) erhaltenen Granulats;
d) Schmieren, sofern ein Schmiermittel vorhanden ist, der getrockneten Granulatkörner und pressen dieser, um den Kern zu erhalten; und
e) Zubereiten einer wässrigen Dispersion, die das pH-abhängige Polymer mit dem Rest der pharmazeutisch akzeptierbaren Vehikel aufweist, und Beschichten des Kerns, um die äußere Schutzschicht und damit die Tablette zu erhalten.

17. Verfahren nach Anspruch 16, bei dem in Schritt b) eine wässrige Lösung aus Polyvinylpyrrolidon zubereitet wird.

18. Verfahren nach Anspruch 16, bei dem in Schritt c) das Trocknen in einem Fluidbett-Trockner durchgeführt wird.

19. Verfahren nach Anspruch 16, bei dem in Schritt d) ein Schmiermittel zugegeben wird, um die getrockneten Granulatkörner zu schmieren, und anschließend das Gemisch gepresst wird, um den Kern zu erhalten.

20. Verfahren nach Anspruch 16, bei dem in Schritt e) eine wässrige Dispersion aus einer alkoholischen Lösung eines Antihaftmittels und des pH-abhängigen Polymers und einem Plastiziermittel zubereitet wird, um den Kern zu beschichten und anschließend die äußere Schutzschicht zu erhalten.

21. Orale pharmazeutische Tablette zur gesteuerten Abgabe von Mesalazin oder eines pharmazeutisch akzeptierbaren Salzes davon nach einem der Ansprüche 1 bis 15 zur Behandlung von ulzerativer Kolitis.

## Revendications

1. Comprimé pharmaceutique oral pour la libération contrôlée de mésalazine ou l'un de ses sels pharmaceutiquement acceptable en tant que principe actif comportant un noyau et un enrobage extérieur gastro-résistant, **caractérisé en ce que** ledit noyau comprend :
i) de la mésalazine de 40 % à 90 % en poids par rapport au poids total du comprimé ; et
ii) une matrice hydrophile composée d'hydroxypropylméthylcellulose (HPMC) ayant une viscosité inférieure à 200 mPa·s dans une solution aqueuse à 2 % et d'hydroxypropylméthylcellulose (HPMC) ayant une viscosité supérieure à 200 mPa·s dans une solution aqueuse à 2 % selon un rapport de poids entre 10:1 et 1:10, ladite matrice hydrophile étant présente de 1 % à 20 % en poids par rapport au poids total du comprimé ; et **en ce que** ledit enrobage extérieur gastro-résistant comprend :
iii) un polymère à libération dépendant du pH,
ledit enrobage extérieur étant présent de 5 % à 25 % par rapport au poids total du comprimé.

2. Comprimé pharmaceutique oral selon la revendication 1, dans lequel la mésalazine est présente de 50 % à 90 %, de préférence de 60 % à 80 % en poids par rapport au poids total du comprimé.

3. Comprimé pharmaceutique oral selon la revendication 1, dans lequel la matrice hydrophile est présente de 1 % à 15 %, de préférence de 2 % à 10 %, et de manière encore préférée de 3 % à 5 % en poids par rapport au poids total du comprimé.

4. Comprimé pharmaceutique oral selon la revendication 1, dans lequel la couche d'enrobage extérieure est présente de 10 % à 20 % par rapport au poids total du comprimé.

5. Comprimé pharmaceutique oral selon la revendication 1, dans lequel la matrice hydrophile est composée d'hydroxypropylméthylcellulose (HPMC) ayant une viscosité inférieure à 200 mPa·s dans une solution aqueuse à 2 % et d'hydroxypropylméthylcellulose (HPMC) ayant une viscosité supérieure à 200 mPa·s dans une solution aqueuse à 2 % selon un rapport de poids de 1:1.

6. Comprimé pharmaceutique oral selon la revendication 1, dans lequel la matrice hydrophile est présente de 1 % à 15 %, de préférence de 2 % à 10 %, et de manière encore préférée de 3 % à 5 % en poids par rapport au poids total du comprimé.

7. Comprimé pharmaceutique oral selon la revendication 1, dans lequel le polymère à libération dépendant du pH est présent dans une quantité allant de 15 % à 75 % en poids de la couche d'enrobage extérieure du comprimé.

8. Comprimé pharmaceutique oral selon la revendication 1, dans lequel le noyau comprend en outre un excipient pharmaceutiquement acceptable choisi parmi un agent de remplissage, un liant, un agent anti-adhérent, un lubrifiant ou un désintégrant.

9. Comprimé pharmaceutique oral selon la revendication 1, dans lequel la couche d'enrobage extérieure du comprimé comprend en outre un excipient pharmaceutiquement acceptable choisi parmi un agent anti-adhérent, un plastifiant ou un colorant.

10. Comprimé pharmaceutique oral selon la revendication 8, dans lequel l'agent de remplissage est présent dans une quantité allant de 0,5 % à 10 %, de préférence de 0,5 % à 8 %, et de manière encore préférée de 1 % à 5 % en poids du poids total du comprimé.

11. Comprimé pharmaceutique oral selon la revendication 8, dans lequel le liant est présent dans une quantité allant de 0,1 % à 10 %, de préférence de 0,5 % à 9 %, et de manière encore préférée de 1 % à 7,5 % en poids du poids total du comprimé.

12. Comprimé pharmaceutique oral selon les revendications 8 et 9, dans lequel l'agent anti-adhérent est présent dans une quantité allant de 0,1 % à 5 %, de préférence de 0,1 % à 3,5 %, de manière encore préférée de 0,1 % à 1,5 % en poids par rapport au poids total du comprimé, et jusqu'à 30 %, de préférence de 5 % à 30 % en poids par rapport au poids total de la couche d'enrobage extérieure.

13. Comprimé pharmaceutique oral selon la revendication 8, dans lequel le lubrifiant est présent de 0,1 % à 5 %, de préférence de 0,1 % à 3 % en poids par rapport au poids total du comprimé.

14. Comprimé pharmaceutique oral selon la revendication 8, dans lequel l'agent désintégrant est présent de 1 % à 10 %, de préférence de 1 % à 8 %, de manière encore préférée de 2,5 % à 7,5 % en poids par rapport au poids total du comprimé.

15. Comprimé pharmaceutique oral à libération contrôlée selon la revendication 9, dans lequel l'agent plastifiant est présent dans une quantité allant jusqu'à 20 %, de préférence de 5 % à 20 % en poids par rapport au poids total de la couche d'enrobage extérieure.

16. Procédé pour obtenir le comprimé pharmaceutique oral selon l'une quelconque des revendications 1 à 15, **caractérisé en ce que** les étapes suivantes sont réalisées :
a) mélanger la mésalazine avec la matrice hydrophile composée d'hydroxypropylméthylcellulose (HPMC) ayant une viscosité inférieure à 200 mPa·s dans une solution aqueuse à 2 % et d'hydroxypropylméthylcellulose (HPMC) ayant une viscosité supérieure à 200 mPa·s selon un rapport de poids entre 1:10 et 10:1, et avec le désintégrant et le liant, s'ils sont présents, pour obtenir une préparation mélangée ;
b) granuler la préparation mélangée obtenue à l'étape a) avec de l'eau ou avec une solution aqueuse de liant, préparée auparavant, quand le liant est présent à l'étape a) ;
c) sécher le granulat obtenu à l'étape b) ;
d) lubrifier, si un lubrifiant est présent, les granules séchés et les comprimer pour obtenir le noyau ; et
e) préparer une dispersion aqueuse qui comprend le polymère dépendant du pH avec le reste des excipients pharmaceutiquement acceptables, et enrober le noyau pour obtenir la couche d'enrobage extérieure, et ainsi le comprimé.

17. Procédé selon la revendication 16, dans lequel à l'étape b) une solution aqueuse de polyvinylpyrrolidone est préparée.

18. Procédé selon la revendication 16, dans lequel à l'étape c) le séchage est réalisé dans un séchoir à lit fluidisé.

19. Procédé selon la revendication 16, dans lequel à l'étape d) un lubrifiant est ajouté pour lubrifier les granules séchés et, ensuite, le mélange est comprimé pour obtenir un noyau.

20. Procédé selon la revendication 16, dans lequel à l'étape e) une dispersion aqueuse d'une solution alcoolique d'un agent anti-adhérent et du polymère dépendant du pH et d'un plastifiant est préparée pour enrober le noyau et ensuite obtenir la couche d'enrobage extérieure.

21. Comprimé pharmaceutique oral pour la libération contrôlée de mésalazine ou l'un de ses sels pharmaceutiquement acceptable selon l'une quelconque des revendications 1 à 15 pour le traitement de la colite ulcéreuse.
